# DEMANDE DE BREVET EUROPEEN

(11) **EP 4 166 067 A1**
(43) Date de publication de la demande: **19.04.2023**
(21) Numéro de dépôt: 21306444.7
(22) Date de dépôt: 15.10.2021
(51) Int. Cl.: A61B 5/00, G06N 3/02, A61B 5/377, A61B 5/383, A61B 5/246, A61B 5/374, G06F 3/01, G16H 50/20

(54) **DISPOSITIF EMBARQUÉ POUR LA COLLECTE SYNCHRONISÉE D'ONDES CÉRÉBRALES ET DE DONNÉES ENVIRONNEMENTALES**

(71) Demandeur: Mentalista, 94800 Villejuif (FR)
(72) Inventeur: DIDIER, Bastien, 94800 Villejuif (FR)
(74) Mandataire: Novagraaf Technologies

(57) **Abrégé**

Dispositif (1) d'analyse automatique d'ondes cérébrales, configuré pour être porté par un utilisateur, comportant
au moins un capteur d'ondes cérébrales (11, 12), fournissant un flux (21, 22) de données cérébrales associées audit utilisateur ;
au moins un capteur environnemental (13, 14), fournissant un flux (23, 24) de données environnementales ;
des moyens (15) pour sélectionner un signal extrait dudit flux de données cérébrales, pour les associer à au moins un signal correspondant extrait dudit flux de données environnementales, pour mémoriser lesdits signaux et pour les transmettre vers un organe de traitement déporté.

## Description

### DOMAINE DE L'INVENTION

La présente invention est relative au domaine de la collecte des ondes cérébrales via des interfaces cerveau-machine. En particulier, elle vise à collecter des ondes cérébrales via des capteurs, et à les associer à des données issues de capteurs environnementaux.

### CONTEXTE DE L'INVENTION

Les interfaces neuronales, ou interfaces cerveau-machine, sont un domaine relativement nouveau de la recherche, mais certains dispositifs sont déjà commercialisés.

La page wikipedia suivante mentionne un état de l'art sur le sujet et quelques applications pratiques prometteuses :
https://fr.wikipedia.org/wiki/Interface_neuronale_directe

On peut par exemple citer l'aide aux personnes handicapées physiques qui peuvent ainsi agir sur leur environnement en utilisant uniquement la pensée.

La société Emotiv Systems commercialise un casque EPOC+ équipé de 14 électrodes électroencéphalographiques (EEG) réparties sur le crâne de l'utilisateur.

Un tel dispositif permet donc d'acquérir des flux de données cérébrales, relatives à l'activité mentale de l'utilisateur. Il est ensuite possible de traiter ces données afin de déclencher une action telle la commande d'un objet, par exemple.

Néanmoins, ce traitement est extrêmement difficile car on ne dispose d'aucun moyen pour associer un signal extrait de ces données cérébrales à une telle valeur sémantique qui pourrait ensuite permettre de déclencher une action appropriée.

L'article « ThroughtViz: Visualizing Human Thoughts Using Generative Adversarial Network » de Praveen Tirurpattur, Concetto Spampinato, Yogesh Singh Rawat et Mubarak Shah, in 2018 ACM Multimedia Conférence on Multimedia Conference, MM 2018, Seoul, Republic of Korea, October 22-26, 2018. pages 950-958, ACM, 2018, représente une tentative de classifier les signaux cérébraux à l'aide d'images perçues par un utilisateur.

Plus précisément, l'article envisage d'utiliser un réseau de neurones artificiels pour permettre une généralisation et l'élaboration d'un modèle prédictif pour déterminer une image à partir de données cérébrales issues d'un électroencéphalogramme (EEG).

Afin de constituer ce modèle prédictif, l'article propose une phase d'apprentissage dans laquelle un sujet est assis devant un écran et regarde une série d'images selon une séquence définie. Lors de l'apparition de chaque image, l'activité cérébrale est échantillonnée et labélisée avec le nom de la classe de l'image. Une fois collectées, les données cérébrales sont utilisées pour l'entraînement de réseaux antagonistes génératifs (GAN pour « *Generative Adversarial Networks* », en anglais). L'objectif est de faire apprendre au modèle constitué par ce réseau des représentations simplifiées de concepts comme « tigre » ou « le nombre 1 », etc.

Cette approche souffre toutefois de plusieurs inconvénients. Notamment ce processus d'apprentissage est très contraignant et ne permet pas de constituer un ensemble d'apprentissage suffisamment pertinent pour permettre la constitution d'un prédicteur efficace et précis.

### RESUME DE L'INVENTION

Un objectif de la présente invention est de fournir un dispositif et une méthode palliant au moins partiellement les inconvénients précités.

Plus particulièrement, selon des modes de réalisation, elle vise à permettre la constitution d'un ensemble d'apprentissage efficace de façon aisée et peu coûteuse par rapport aux solutions de l'état de la technique.

À ces fins, l'invention se base sur un dispositif adapté pour être porté par un utilisateur et disposant à la fois d'au moins un capteur d'ondes cérébrales et au moins un capteur environnemental.

L'apport de ce capteur environnemental et la combinaison de ces deux sortes de capteurs permettent notamment des fonctionnalités nouvelles et, selon un mode de réalisation, de substantiellement améliorer un mécanisme d'apprentissage d'un réseau de neurones.

En particulier, selon un premier aspect, la présente invention peut être mise en œuvre par un dispositif d'analyse automatique d'ondes cérébrales, configuré pour être porté par un utilisateur, comportant
au moins un capteur d'ondes cérébrales, fournissant un flux de données cérébrales associées audit utilisateur ;
au moins un capteur environnemental, fournissant un flux de données environnementales ;
des moyens pour sélectionner un signal extrait dudit flux de données cérébrales, pour les associer à au moins un signal correspondant extrait dudit flux de données environnementales, pour mémoriser lesdits signaux et pour les transmettre vers un organe de traitement déporté.

Suivant des modes de réalisation préférés, l'invention comprend une ou plusieurs des caractéristiques suivantes qui peuvent être utilisées séparément ou en combinaison partielle entre elles ou en combinaison totale entre elles :
- au moins un capteur environnemental comprend une caméra vidéo, orientée de sorte à capturer une zone de l'espace correspondant substantiellement à celle capturée par le regard dudit utilisateur ;
- au moins un capteur environnemental comprend un capteur sonore ;
- le dispositif comporte en outre un serre-tête prévu pour maintenir ledit dispositif en place sur la tête dudit utilisateur et pour maintenir lesdits capteurs d'ondes cérébrales en contact ou à proche vicinité de ladite tête ; et un bloc d'alimentation permettant le fonctionnement desdits capteurs et desdits moyens ;

- le dispositif est configuré pour associer auxdits signaux un horodatage et/ou un identifiant dudit dispositif ;
- la localisation d'un capteur cérébral dépend d'un type dudit au moins capteur environnemental ;
- le dispositif comporte en outre des moyens pour acquérir un flux de données externes provenant d'une source extérieure audit dispositif, et pour associer ledit signal extrait dudit flux de données cérébrales à au moins un signal correspondant extrait dudit flux de données externes.

Selon un second aspect, l'invention peut également être mise en œuvre par un système comportant un ou plusieurs dispositifs tel que précédemment défini, ainsi qu'un organe de traitement déporté configuré pour mémoriser l'ensemble des signaux provenant dudit un ou plusieurs dispositifs.

Suivant des modes de réalisation préférés, l'invention comprend une ou plusieurs des caractéristiques suivantes qui peuvent être utilisées séparément ou en combinaison partielle entre elles ou en combinaison totale entre elles :
- le système comporte des moyens pour extraire une information de classification à partir desdites données, et pour constituer un ensemble associant lesdites données à l'information de classification extraite ;
- le système comporte des moyens pour entraîner un réseau de neurones sur la base dudit ensemble.

Selon un autre aspect, l'invention peut également être mise en œuvre par un procédé d'analyse automatique d'ondes cérébrales à l'aide d'un dispositif configuré pour être porté par un utilisateur, le procédé comportant des étapes de :
- génération d'un flux de données cérébrales associées audit utilisateur au moyen d'au moins un capteur d'ondes cérébrales ;
- génération d'un flux de données environnementales au moyen d'au moins un capteur environnemental ;
- association d'un signal sélectionné extrait dudit flux de données cérébrales à au moins un signal correspondant extrait dudit flux de données environnementales ;
- mémorisation desdits signaux et transmission vers un organe de traitement déporté.

Suivant des modes de réalisation préférés, l'invention comprend une ou plusieurs des caractéristiques suivantes qui peuvent être utilisées séparément ou en combinaison partielle entre elles ou en combinaison totale entre elles :
- le procédé comporte en outre une étape d'extraction d'une information de classification à partir desdites données, et une étape de constitution d'un ensemble associant lesdites données à l'information de classification extraite.
- le procédé comporte en outre une étape d'entraînement d'un réseau de neurones sur la base dudit ensemble.
- le procédé comporte en outre une étape de prédiction d'une information de classification à partir d'au moins un nouveau signal extrait des données cérébrales.

Selon un autre aspect, l'invention peut également être mise en œuvre par un programme d'ordinateur comportant des instructions pour mettre en œuvre le procédé tel que précédemment décrit lorsque celui-ci est exécuté sur une ou plusieurs plateformes de traitement de l'information.

D'autres caractéristiques et avantages de l'invention apparaîtront à la lecture de la description qui suit d'un mode de réalisation préféré de l'invention, donnée à titre d'exemple et en référence aux dessins annexés

### BRÈVE DESCRIPTION DES FIGURES

Les dessins annexés illustrent l'invention :
La figure 1 schématise un dispositif selon un mode de réalisation de l'invention.
La figure 2 illustre un exemple d'arrangement fonctionnel d'un dispositif selon un mode de réalisation de l'invention.
La figure 3 illustre, sous forme d'organigramme, un procédé selon un mode de réalisation de l'invention.

### DESCRIPTION DÉTAILLÉE DE MODES DE RÉALISATION DE L'INVENTION

Un aspect de l'invention est relatif à un dispositif d'analyse automatique d'ondes cérébrales configuré pour être porté par un utilisateur.

Différents modes de réalisation d'un tel dispositif. Par exemple, ce dispositif peut être un casque ou un bandeau que l'utilisateur doit porter.

Selon un mode de réalisation, le dispositif possède un serre-tête prévu pour maintenir le dispositif en place sur la tête de l'utilisateur et pour maintenir les capteurs d'ondes cérébrales en contact ou à proche vicinité de la tête ; et un bloc d'alimentation permettant le fonctionnement des capteurs et des moyens de traitement.

Ce dispositif comporte également au moins un capteur d'ondes cérébrales, fournissant un flux de données cérébrales associées à l'utilisateur qui le porte, au moins un capteur environnemental, fournissant un flux de données environnementales et des moyens de traitement de ces flux de données.

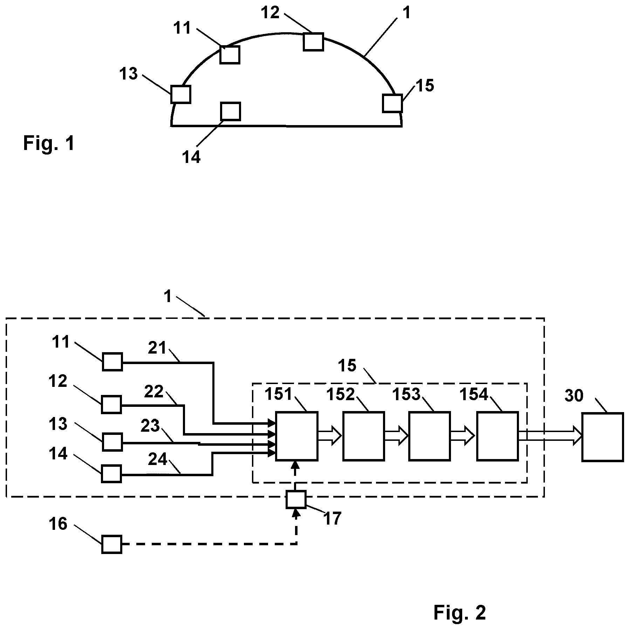

La figure 1 schématise un tel dispositif selon un mode de réalisation de l'invention.

Le dispositif 1 comporte des capteurs d'ondes cérébrales 11, 12 qui peuvent être de différents types, selon les technologies disponibles. Les différentes techniques d'acquisition des ondes cérébrales comprennent l'électroencéphalographie (EEG), l'imagerie spectroscopique proche infrarouge fonctionnelle (fNIRS pour « *functional near-infrared spectroscopy* » en anglais), la magnétoencéphalographie (MEG), mais d'autres techniques pourraient être utilisées pour récupérer l'activité des neurones.

En outre, deux capteurs sont indiqués pour la clarté de la figure, mais de nombreux capteurs peuvent être positionnés à différents endroits du dispositif 1 correspondant à des zones diverses du cerveau. Un grand nombre de capteurs permet de capturer l'activité cérébrale de façon plus globale, dans la mesure où un capteur ne peut acquérir efficacement que l'activité d'une zone limitée autour de son emplacement et que les différentes parties du cerveau ont des activités spécialisées (cortex auditif, cortex visuel, etc.)

Selon un mode de réalisation de l'invention, la localisation des capteurs peut dépendre du type de capteurs environnementaux utilisés.

Ainsi, si un des capteurs environnementaux est un capteur vidéo, on peut positionner au moins un capteur cérébral (ou électrode) au niveau du cortex visuel. Le cortex visuel couvre le lobe occipital, sur les faces latérales et internes, et empiète sur le lobe pariétal et le lobe temporal. Il est chargé de traiter les informations visuelles. L'étude du cortex visuel en neurosciences a permis de le découper en une multitude de sous-régions fonctionnelles (V1, V2, V3, V4, MT, etc.) qui traitent chacune ou collectivement des multiples propriétés des informations provenant des voies visuelles (formes, couleurs, mouvements, etc.). Il peut aussi être intéressant de positionner plusieurs capteurs afin de recueillir des données cérébrales relatives à ces différentes activités du cerveau.

Si un des capteurs environnementaux est un capteur sonore, c'est-à-dire un microphone, au moins un capteur cérébral peut être positionné au niveau du cortex auditif. Le cortex auditif est la partie du cerveau qui analyse les informations auditives, c'est-à-dire les informations extraites des sons par l'ouïe. Il occupe la partie supérieure du lobe temporal.

Si un des capteurs environnementaux est un capteur de mouvement, c'est-à-dire un dispositif d'enregistrement des positions et rotations des membres et du corps, au moins un capteur cérébral peut être positionné au niveau du cortex moteur. Le cortex moteur est la partie du cerveau qui participe à la planification, au contrôle et à l'exécution des mouvements des muscles du corps. Il est situé dans la partie postérieure du lobe frontal.

Si un des capteurs environnementaux est un capteur capacitif, c'est-à-dire un dispositif d'enregistrement de la pression, par exemple du toucher des doigts, au moins un capteur cérébral peut être positionné au niveau du cortex somatosensoriel. Le cortex somatosensoriel est la partie du cerveau qui reçoit les informations en provenance de la surface du corps. Il est situé dans la partie antérieure du lobe pariétale.

Il y a ainsi un couplage entre le type des capteurs environnementaux et la localisation des capteurs cérébraux. Comme on le verra plus loin, la combinaison de la présence des capteurs environnementaux et du positionnement d'un capteur cérébral permet un effet synergique d'une meilleure contextualisation de l'information récupérée.

Les capteurs environnementaux sont des capteurs visant à récupérer de l'information sur l'environnement de l'utilisateur du dispositif. Cet environnement se définit par ce qui entoure cet utilisateur. Les éléments qui entourent l'utilisateur peuvent se manifester à lui, et donc influer sur son comportement mental, de différentes façons : par la vue, par le son (un élément produit du bruit, du son...), etc.

Comme on le verra plus loin, le rôle de ces capteurs environnementaux est d'apporter de l'information pertinente pouvant être mise en correspondance avec les données cérébrales, et pouvant ainsi, selon un mode de réalisation, servir à contextualiser ces dernières. D'une façon générale, les données issues des capteurs environnementaux servent à expliquer les données cérébrales qui, seules, peuvent être difficilement interprétables.

Selon un mode de réalisation de l'invention, au moins un capteur environnemental peut être une caméra vidéo, orientée de sorte à capturer une zone de l'espace correspondant substantiellement à celle capturée par le regard de l'utilisateur. En d'autres termes, cette caméra est orientée vers l'avant du dispositif 1. L'angle de prise de vue peut être configuré de sorte à capturer l'angle de vue d'un utilisateur en prenant en compte les mouvements habituels de l'œil.

D'autres caméras peuvent être positionnées et orientées différemment afin de multiplier les points de vue de l'environnement de l'utilisateur.

Selon un mode de réalisation, une caméra peut être orientée vers le visage de l'utilisateur afin de capturer ses micromouvements faciaux et/ou les mouvements de ses pupilles.

Différentes technologies de caméras vidéos peuvent être utilisées. Il peut s'agir de capteurs CCD (pour « *Charge Coupled Device* » en anglais), CMOS, infrarouges..., couplés à différents dispositifs optiques (lentilles...). Il est possible d'envisager une caméra à 360° par exemple.

Selon un mode de réalisation, au moins un capteur environnemental est un capteur sonore. Ce capteur peut typiquement être un capteur de type MEMS (pour « *Micro-Electro-Mechanical System* » en anglais). Le positionnement des capteurs sonores peut varier en fonction de la nature du son à acquérir. Plusieurs capteurs peuvent être positionnés de sorte à capturer l'environnement sonore de la façon la plus complète possible. Par exemple, deux capteurs peuvent être positionnés de chaque côté du dispositif, afin de capturer l'environnement humain à la façon des oreilles humaines.

D'autres capteurs environnementaux possibles comprennent un gyroscope, un dispositif de localisation en temps-réel (RTLS pour « *Real-Time Location System* » en anglais), des capteurs corporels pour l'acquisition de données de pulsation corporelle, de température, de sudation, etc., des capteurs de données météorologiques, de données colorimétriques, des LIDAR, etc.

Tous ces capteurs fournissent un flux de données environnementales qui sont transmises aux moyens de traitement 15 du dispositif 1.

Par ailleurs, selon un mode de réalisation de l'invention, le dispositif comporte en outre des moyens pour acquérir un flux de données externes provenant d'une source extérieure audit dispositif.

Ces moyens peuvent comprendre une interface radio et des moyens logiciels et matériels pour communiquer avec cette source extérieure et effectuer les traitements de signaux appropriés.

Cette source extérieure peut être une caméra vidéo, par exemple une caméra de vidéo-surveillance. Cette caméra vidéo peut apporter des informations supplémentaires par rapport à celle(s) présente(s) dans le dispositif 1 en apportant une vue extérieure globale, sans doute plus vaste mais en tout cas différente, de celles acquises du point de vue de l'utilisateur via le dispositif 1.

De la même façon, cette source extérieure peut être un capteur sonore.

La source peut être une source de données météorologiques, etc.

Certaines données externes peuvent être acquises grâce à des services distants, accessibles le plus souvent sous forme d'API (« *Application Programming Interface* ») et de requêtes sur un réseau (Internet par exemple).

Le flux de données externe est transmis aux moyens de traitement 15 du dispositif, de la même façon que les flux de données environnementales.

Le dispositif 1 comporte donc également des moyens de traitement.

Ces moyens de traitement permettent de traiter les flux de données et de les exploiter.

Plus particulièrement, ces moyens 15 sont configurés pour
- sélectionner un signal extrait du flux de données cérébrales,
- les associer à au moins un signal correspondant extrait du flux de données environnementales (et externes, selon un mode de réalisation),
- mémoriser ces signaux et
- les transmettre vers un organe de traitement déporté.

Ces moyens de traitement sont typiquement mis en œuvre par un ou plusieurs microprocesseurs, une ou plusieurs mémoires et des composants spécifiques divers, ainsi que par des modules logiciels permettant au(x) microprocesseur(s) d'assurer les fonctionnalités indiquées ci-dessus.

En particulier, selon un mode de réalisation, les fonctions de traitement haut-niveau comme la sélection et l'association peuvent être mise en œuvre par un premier moyen, ou circuit, tandis que la mémorisation est mise en œuvre par un moyen, ou circuit, de mémorisation (comportant essentiellement une mémoire et des circuits de gestion de cette mémoire), et la transmission peut être assurée par des circuits de communication.

Les circuits de communication peuvent permettre la connexion à différents types de réseau d'accès : réseaux cellulaires, notamment de 4^{e} ou 5^{e} génération, réseaux locaux de type WLAN ou WIFI, ou de proximité de type Bluetooth ou NFC (Near Field Communication), etc.

Le terme « circuit » est compris dans la présente demande comme comprenant des éléments matériels éventuellement associés à des éléments logiciels dans la mesure où certains éléments matériels peuvent être programmés. Notamment, le terme circuit comprend des mises en œuvre purement matérielles, sous forme de circuits numériques ou analogues imprimés spécifiquement, des mises en œuvre basées, entièrement ou partiellement, sur des éléments de type microprocesseur ou processeur, qui font l'objet d'une programmation par des instructions logicielles stockées dans une ou plusieurs mémoires associées, etc. Les instructions logicielles peuvent consister uniquement aux instructions nécessaires pour les opérations de base des processeurs (le « *firmware* ») tandis que les instructions logicielles nécessaires à la réalisation des fonctions des modes de réalisation de l'invention peuvent être stockées soit dans ces mêmes mémoires associées aux processeurs, soit dans des mémoires déportées. Dans ce dernier cas, ces instructions logicielles ne sont présentes dans le circuit que lorsque le circuit est en opération pour réaliser les fonctions selon les modes de réalisation de l'invention.

Le dispositif comporte également des composants nécessaires pour fonctionner de façon autonome. Il peut notamment comporter un bloc d'alimentation. Celui-ci peut comprendre une batterie rechargeable. Les moyens susmentionnés comprennent des antennes pour assurer sa connectivité, des mécanismes de stockage (mémoires).

Selon un mode de réalisation de l'invention, le dispositif peut comprendre des moyens de retour haptique (moteur vibrant, ostéophonie...) afin d'améliorer l'expérience pour l'utilisateur et lui faire comprendre les processus enclenchés par le dispositif.

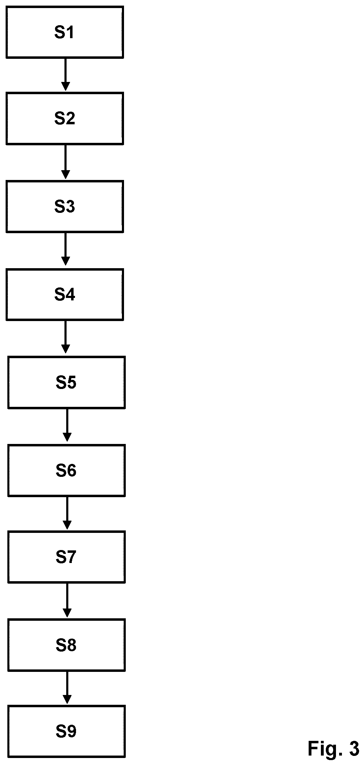

La figure 2 illustre un exemple d'arrangement fonctionnel d'un dispositif selon un mode de réalisation de l'invention. Cette figure va être décrite conjointement avec la figure 3 qui illustre un procédé selon un mode de réalisation de l'invention.

Deux capteurs d'ondes cérébrales 11, 12 sont représentés. Dans une étape S1 sur la figure 3, ils fournissent un flux de données, respectivement 11, 12, destiné aux moyens de traitement 15.

Ce flux de données cérébrales peut être structuré de différentes façons et le format des données elles-mêmes peut également prendre plusieurs formes.

Deux capteurs environnementaux 13, 14 sont représentés. Dans une étape S2, ils fournissent un flux, respectivement 23, 24, de données environnementales à destination des moyens de traitement 15.

Également, selon un mode de réalisation de l'invention, une source extérieure 16 peut transmettre un flux de données externe 26 à des moyens particuliers 17 (interface radio, moyens de prétraitement, etc.), configurés pour les acquérir et retransmettre vers les moyens de traitement 15.

Les moyens de traitement 15 peuvent se subdiviser en plusieurs modules fonctionnels 151, 152, 153, 154. Comme décrit précédemment, ces modules peuvent être des composants matériels distincts, ou des modules logiciels distincts, ou une combinaison des deux.

Dans un premier temps, les flux reçus des différents capteurs sont mémorisés par des moyens de mémorisation 151.

Il n'est pas mentionné ici un module de prétraitement dont l'objet serait de mettre en forme les données reçues dans un format uniforme et adapté pour les traitements ultérieurs. Il peut notamment s'agir de filtrage, de mise à l'échelle, etc. Ces traitements sont classiques en soit et directement accessibles par l'homme du métier, connaisseur de l'acquisition de données brutes issues de capteurs variés en vue de leur traitement numérique.

Puis, dans une étape S3, le contenu de la mémoire est analysé par un module d'association 152 afin d'associer, ou mettre en correspondance ou encore « apparier », du contenu des données cérébrales avec du contenu des données environnementales (et des données externes si elles existent).

Cette association se fait par paquets de données. Les moyens de mémorisation 151 sont donc prévus pour permettre de mémoriser au moins un paquet de données (pour chaque flux) afin de permettre leur association en bloc par les moyens d'association.

Plus précisément, on peut sélectionner un paquet, ou signal, dans le flux de données cérébrales, et associer ce signal à un signal « correspondant » dans les différents flux de données environnementales (et externes s'ils existent).

Un signal (ou paquet) contient donc des données extraites des flux. Dans la suite, les termes « signal » et « données » pourront, selon le contexte, être utilisés de façon interchangeable, le terme « données » pouvant sous-entendre « données contenues dans le signal ».

Un paquet, ou signal, peut comprendre les données contenues dans une fenêtre temporelle, dont la taille peut être définie par les besoins des applications. Cette taille peut être déterminée par des algorithmes dynamiques (par exemple la reconnaissance d'un objet dans des données visuelles ou auditives : leur reconnaissance détermine la fin de la fenêtre temporelle et « instancie » ainsi le paquet, ou signal.

De même, en fonction des applications, les signaux peuvent ne contenir qu'une partie des données obtenues des capteurs, ou peuvent contenir des données prétraitées (c'est-à-dire ayant fait l'objet d'un prétraitement des données brutes, tels qu'un filtrage, etc.)

Par ailleurs, l'acquisition des données peut se faire en continu à chaque itération du programme mis en place par les moyens de traitement 15 et les capteurs sont échantillonnés et synchronisés.

Selon un mode de réalisation, un événement déclencheur peut être utilisé pour que le dispositif ne relève pas les données en continu. L'événement déclencheur peut être de différentes natures : la détection d'un visage, d'un bruit particulier, d'un type d'onde cérébrale... Une fois le déclencheur ciblé, les capteurs sont échantillonnés et synchronisés jusqu'à ce que l'état ne réponde plus au critère de ciblage.

Selon un mode de réalisation, pour chaque signal du flux de données cérébrales, on lui associe l'ensemble des signaux correspondants extraits des flux environnementaux (et externes s'ils existent). Selon un autre mode de réalisation, uniquement une partie des signaux extraits des flux environnementaux (et externes) peuvent être associés.

Dans la mesure où plusieurs capteurs d'ondes cérébrales existent, l'association peut se faire pour chaque flux individuel séparément, ou en regroupant les flux issus des différents capteurs en un seul flux global. Dans ce dernier cas, l'association peut être effectuée sur ce flux global.

Toutefois, ces différentes mises en œuvre ne représentent que des détails de mise en œuvre et reviennent fonctionnellement au même puisque ce qui compte est d'associer les données cérébrales aux données environnementales, quelque soit la façon dont ces données sont organisées et ensuite associées. Il est en effet facile de convertir une structure de données en un autre, en fonction des applications subséquentes.

Une fois associés, les signaux peuvent être mémorisés, dans une étape S4, par un module de mémorisation 153, au sein d'une mémoire locale.

En particulier, les ondes cérébrales, exprimées par des valeurs numériques acquises, sont ajoutées aux valeurs des autres capteurs dans une structure de données permettant de les regrouper par paquets.

Chaque type de données peut être stocké de différentes façons, notamment en fonction de ses spécificités.

Par exemple, les informations cérébrales peuvent être stockées par
- Canaux (c'est-à-dire par capteurs),
- Ensemble de canaux,
- Séries temporelles,
- Spectrogrammes, etc.

Les données visuelles peuvent par exemple être stockées sous forme
- d'images,
- de séquences d'images,
- de vidéos, etc.

Les données sonores peuvent par exemple être stockées sous forme
- d'échantillons,
- de flux ou « *stream* » en anglais, etc.

Ces signaux peuvent par ailleurs être associés à un horodatage et/ou à un identifiant du dispositif 1

Selon un mode de réalisation, toutes ces données peuvent être stockées dans un tableau associant un signal extrait d'un flux de données cérébrales, un ou plusieurs signaux extraits des flux de données environnementales (et externes s'il y a lieu) et un horodatage.

Par « tableau », on peut entendre une association d'une clé à une valeur. Un tableau peut avoir différentes clés en fonction du contexte auquel sont associées des valeurs.

Un exemple d'un tel tableau est donné ci-dessous à titre purement illustratif.

| | | | | |
|---|---|---|---|---|
| Signal cérébral | Signal environnemental 1 | Signal environnemental 2 | Horodatage | Identifiant |
| | | | | |

Ces données peuvent éventuellement être hachées à des fins de sécurisation des données.

À titre d'exemple, dans une application où on chercherait à associer des ondes cérébrales d'une personne avec les données visuelles de son environnement lorsqu'une machine à café est présente dans celui-ci, le paquet transmit pourrait être instancié à la détection de l'objet dans l'environnement de l'utilisateur grâce à une caméra et à un algorithme de reconnaissance d'objet.

Dans cet exemple, ce paquet transmet une vue instantanée contenant, au moins :
- l'identifiant de l'utilisateur,
- l'identifiant de l'appareil
- l'horodatage
- les données de configuration de l'appareil
- les données brutes de la caméra
- les données de localisation et de segmentation de l'objet dans l'image
- les enregistrements des ondes cérébrales.

Selon un mode de réalisation, un temps de latence peut être prévu afin de décaler temporellement le signal cérébral avec les signaux environnementaux correspondants. En effet, la réalité biologique impose des temps de transmission et de traitement de l'information. Par exemple, une image qui arrive à la rétine met quelques millisecondes avant d'impacter les neurones. Ce décalage peut être pris en compte par le dispositif.

Un module 154 est configuré pour les transmettre vers un organe de traitement déporté 30. Cette transmission correspond à une étape S5 sur la figure 3.

Cette transmission peut être effectuée au fil de l'eau, sur requête de cet organe déporté 30, de façon périodique, ou selon tout autre mode de réalisation possible permettant la communication des données associées récoltées par le dispositif 1.

Cet organe déporté 30 peut ainsi acquérir un grand nombre de données de la part du dispositif 1. Ces données associent comme on l'a vu des données d'ondes mentales avec des données environnementales. La nature même de ces données « associées » peut permettre de mettre en œuvre un grand nombre d'applications et de services par cet organe déporté, notamment dans un but de prédire une action à effectuer à partir d'au moins un signal nouveau extrait d'un nouveau flux de données cérébrales.

D'une façon générale, cette association permet de mieux comprendre le contexte environnemental associé à des données mentales. Elle permet d'enrichir les interfaces cerveau-machine en y apportant ces informations de contextes et donc d'améliorer l'interprétation par un ordinateur des données mentales.

Cette association, qui contextualise les ondes cérébrales, peut permettre, par exemple, la personnalisation en temps réel d'un modèle de classification permettant à un utilisateur de contrôler une action sur des objets interactifs de son environnement.

Par exemple, de la même façon que certains objets peuvent être pilotés via des gestes détectés par une caméra, un mode de réalisation de l'invention peut permettre de piloter des objets via un pensée instanciée par un signal d'ondes cérébrales.

En outre, cette association permet de constituer une base de données d'ondes cérébrales contextualisées, c'est-à-dire associées à des données environnementales.

Cette base de données peut notamment permettre de créer un ensemble d'apprentissage pour la constitution d'un modèle prédictif.

Une fois un ensemble d'apprentissage constitué, on peut entraîner le modèle prédictif. Une fois correctement entraîné, un tel modèle prédictif permet de généraliser les informations acquises, et permet de prédire des données inconnues sur la base d'un modèle élaboré sur la base d'un ensemble d'apprentissage aussi important et diversifié que possible.

Grâce à l'invention, le modèle prédictif est entraîné sur la base de données cérébrales et environnementales, et il peut en particulier généraliser une relation entre ces deux types de données.

Les finalités d'un tel modèle prédictif comprennent notamment le contrôle d'objets par la pensée, la surveillance des états mentaux d'un individu ou d'un groupe d'individus, etc. D'une façon générale, il permet de déterminer une action à effectuer à partir d'au moins un signal nouveau extrait d'un nouveau flux de données cérébrales.

Il est également à noter, que l'ensemble d'apprentissage se base sur des informations issues de la perception d'un objet réel et non pas, comme dans l'article « ThoughtViz: Visualizing Human Thoughts Using Generative Adversarial Network » précédemment cité, sur des images affichées sur un écran et représentant ces objets.

Or, il apparaît aux inventeurs que la différence entre la perception de la représentation d'un objet et la perception de l'objet physique lui-même induit un biais cognitif qui crée une différence suffisante pour invalider l'applicabilité de l'expérience hors d'un contexte expérimental.

Hors d'un contexte académique et dans le cas concret d'expériences de contrôle mental, par exemple, l'entraînement sur des objets réels, et non sur des représentations de ces objets, permet une amélioration notable des performances du système.

Par ailleurs, il favorise un sentiment d'appropriation beaucoup plus fort pour l'utilisateur, et est en outre beaucoup moins contraignant à mettre en place puisqu'il suffit de se promener et de se confronter à des objets pour, de façon automatique et transparente, permettre l'accumulation de données cérébrales et environnementales, tandis que le dispositif de l'article suscité oblige à rester assis devant un écran pour une acquisition de données beaucoup plus fastidieuse. Il en résulte une génération d'un ensemble de données beaucoup plus important et de façon plus rapide (outre le fait que les données sont enrichies par l'apport des capteurs environnementaux).

Ainsi, le dispositif permet de résoudre le premier problème de la prise en compte d'un ensemble de données non-cérébrales contextualisant les données cérébrales acquises et permettant donc de leur donner du sens.

Ainsi, pour un individu regardant un chat, on pourra interpréter les données cérébrales comme liées à un chat ou à ce chat.

Le dispositif selon l'invention permet également la constitution d'un ensemble d'apprentissage, qui est basé sur des données réelles, suffisamment vaste et donc facile et peu coûteux en investissement.

Selon un mode de réalisation de l'invention, des données associées issues de différents dispositifs peuvent être utilisées.

Cette mise en réseau est possible dans le cadre d'un même organe de traitement déporté auquel sont connectés plusieurs dispositifs. Elle est également possible au niveau des dispositifs eux-mêmes qui peuvent soit communiquer entre eux en mode « pair à pair » soit disposer d'une interface permettant de se connecter sur un réseau afin de partager leurs données.

Dans un tel cadre de mise en réseau, il peut être utile que les données soient associées à un identifiant du dispositif les ayant générées.

Quel que soit le moyen de mise en réseau, celle-ci peut être utile afin d'obtenir des informations plus globales sur les activités mentales de différents utilisateurs. Notamment, il devient ainsi possible de rapprocher des activités mentales d'utilisateurs confrontés à des données environnementales similaires. Cet aspect sera davantage détaillé plus loin, en rapport avec l'extraction d'informations de classification.

Par exemple, la mise en réseau permet de recueillir les données cérébrales de personnes regardant un même objet en même temps. Cette fonctionnalité permet d'étudier les réactions sociales des individus en réaction à un stimuli visuel, sonore, etc.

Il est également possible, de mettre en commun des données effectuées en différentes localisations géographiques, par l'utilisation de réseaux de communication longue-distance (Internet...). Par exemple, on peut recueillir les données cérébrales de personnes regardant une même émission télévisée, afin d'étudier leurs réactions en temps-réel pendant la diffusion. Il est ainsi possible d'influer le déroulement de cette émission en fonction d'une analyse de ces réactions.

Dans une étape S6, sur la figure 3, on extrait une information de classification à partir des données recueillies (cérébrales et environnementales).

Cette étape peut être mise en œuvre par un organe de traitement déporté. Cet organe déporté peut être un équipement personnel de type ordinateur, tablette, terminal de communication de type « smartphone », etc., mais il peut également s'agir d'un service fourni par un serveur ou un groupe de serveurs organisés en ferme ou abstractisés sous forme de plateforme en nuage (ou « *cloud* »).

Dans un autre mode de réalisation, cette étape est mise en œuvre par des moyens de traitement du dispositif lui-même.

Ces informations de classification sont des informations permettant de former des ensembles de signaux associés à l'étape S3, qui sont extraites de ces données elles-mêmes, contenues dans les signaux.

Ces informations de classification doivent être déterminées afin que ces ensembles soient cohérents et pertinents, par exemple en vue d'une application donnée.

Autrement dit, le but de cette étape est de classifier les couples signaux cérébraux/signaux environnementaux, afin de pouvoir constituer des ensembles regroupant les signaux appartenant à une même classe. Elle vise donc à, à la fois, obtenir une bonne qualité de la classification (chaque signal associé est effectivement mis dans la bonne classe) et avoir des classes qui soient pertinentes par rapport à une application donnée ou plus généralement qui aient sémantiquement une signification pour un être humain.

Il s'agit donc d'analyser les données recueillies afin de déterminer des classes pertinentes pour ces regroupements. Typiquement, ces classes sont relatives à un concept sémantique qui permet de contextualiser les données cérébrales.

Il est à noter que cette étape S6 peut être mise en œuvre à différents moments, et ne pas être obligatoire en séquence avec l'étape S5, dans le cas où elle est mise en œuvre directement par le dispositif 1.

Par exemple, le dispositif (ou les dispositifs, dans le cas d'une mise en réseau) peut engranger un grand nombre de données avant que l'étape S6 d'extraction d'information de classification et les étapes subséquentes soient déclenchées. Alternativement, elle peut être déclenchée dès la fin des étapes S4, S5, de mémorisation et transmission des signaux ou après l'étape d'association S3, etc.

Plus précisément, à titre d'exemples, l'extraction des informations de classification peut intervenir :
Au moment de l'acquisition d'une donnée par un capteur (par exemple, une caméra qui cherche un visage donné ou appartenant à une classe donnée) ;
Au moment de l'enregistrement des données (par exemple, on traite le signal audio pour reconnaître des énonciations de l'utilisateur, et on stocke le son brut avec les éventuelles énonciations reconnues par l'appareil) ;

*A posteriori,* sur le serveur, lors d'une phase de recherche non prévue au moment de la collecte (par exemple, des données ont été enregistrées depuis plusieurs semaines, dans un lieu donné, et on cherche ensuite à créer des ensembles cohérents en regroupant les échantillons d'ondes cérébrales par classes).

Dans un mode de réalisation de l'invention, ces informations de classification fournissent directement des étiquettes afin d'entraîner un réseau de neurones (ou tout autre modèle prédictif).

Ces informations de classification peuvent aussi être utilisées pour effectuer des recherches. Par exemple, il devient facile de faire une recherche par mot-clé (« miaulement », « pull vert », « bruit de chantier », « personne blonde », « banane », ...): ces mots clés sont recherchés parmi les informations de classification, et l'on peut ainsi récupérer l'ensemble des associations données cérébrales / données environnementales correspondantes. On peut ainsi comprendre l'activité du cerveau humain dans un certain contexte très précis, indiqué par les mots-clés, notamment en comparant les différentes ondes cérébrales d'un grand nombre de personnes.

Une telle étude n'était pas directement possible avec les systèmes de l'état de la technique du fait de l'impossibilité de recueillir des données cérébrales en grand nombre et nativement associées à des données environnementales permettant de les contextualiser.

Dans un mode de réalisation, les informations de classification sont déterminées à partir des données environnementales (et/ou des données externes).

Par exemple, les données vidéos peuvent être exploitées afin de déterminer des visages.

Pour ce faire, tout ou partie des signaux environnementaux de type « vidéo » peuvent être analysés afin d'y déterminer la présence d'un visage. Pour chaque signal environnemental dans lequel un visage est déterminé, on peut récupérer le signal cérébral associé, ainsi que les éventuels autres signaux environnementaux et externes. Ces différents signaux peuvent ensuite être associés à une information de classification correspondante (par un mot-clé « visage » ou tout autre identifiant approprié).

Le processus de classification peut être plus fin et déterminer des classes différentes pour les visages masculins et féminins, par exemple, ou des visages souriants, etc.

Le même procédé peut être appliqué à d'autres objets qui peuvent être également reconnus dans une image.

Différentes techniques de reconnaissance d'objets (y compris de visages) peuvent être utilisées. Notamment différentes techniques d'intelligence artificielle peuvent être exploitées comme les réseaux de neurones ou les machines à vecteurs de support (SVM, pour « *Support Vector Machine »* en anglais).

De la même façon, les données sonores peuvent être exploitées afin de déterminer des sons particuliers.

Pour ce faire, tout ou partie des signaux environnementaux de type « audio » ou « son », peuvent être analysés afin d'y déterminer la présence d'un son identifiable (bruit de moteur, paroles intelligibles, musique...). Pour chaque signal environnemental dans lequel un tel son est déterminé, on peut récupérer le signal cérébral associé, ainsi que les éventuels autres signaux environnementaux et externes. Ces différents signaux peuvent ensuite être associés à une information de classification correspondante (par un mot-clé « chanson » ou tout autre identifiant approprié).

Là encore, l'analyse peut être plus fine, en définissant des classes différentes en fonction, par exemple, de la reconnaissance de la musique (compositeur, interprète, type de musique, etc.), d'une donnée sémantique déterminée à partir de paroles intelligibles, etc.

Dans ce dernier cas, un mécanisme de type STT (« *Speech To Text* ») peut être utilisé afin de détecter ces mots ou des phrases prononcés par l'utilisateur ou par des personnes autour de lui.

Un autre exemple est l'utilisation de la position de l'utilisateur.

Pour ce faire, les signaux environnementaux issus d'un capteur de position sont analysés et des informations de classification sont déterminées à partir de ces localisations. En particulier, les classes peuvent être déterminées en fonction de la répartition des localisations acquises dans l'ensemble des données disponibles. Ainsi, la zone géographique correspondant à chaque classe peut être dynamiquement et automatiquement adaptée aux signaux disponibles, de sorte à former des classes de cardinal non nul et non trop important.

Pour chaque signal environnemental correspondant à une même information de classification, on peut récupérer le signal cérébral associé, ainsi que les éventuels autres signaux environnementaux et externes. Ces différents signaux peuvent ensuite être associés à une information de classification correspondante (par un mot-clé identifiant le lieu, une coordonnée GPS, ou tout autre identifiant approprié).

On peut ainsi créer des classes regroupant les signaux cérébraux des différentes personnes présentes en un même lieu. Il est alors aisé de récupérer ces informations par une simple interrogation avec des coordonnées, ce qui ouvre la porte à un grand nombre de nouvelles applications. Une seconde étape d'extraction peut être envisagée pour identifier les personnes qui ont regardé les mêmes choses dans ce lieu.

Selon un mode de réalisation, les informations de classification sont déterminées à partir des données cérébrales.

Par exemple, on peut rechercher des motifs particuliers au sein des signaux cérébraux.

Une possibilité est de rechercher des motifs alpha. Les activités électriques cérébrales rythmiques chez l'être humain sont classées selon leur fréquence. Le rythme alpha désigne un rythme cérébral, c'est-à-dire une oscillation électroencéphalographique (EEG) résultant de l'activité électrique du cerveau, dont la fréquence est comprise, substantiellement, entre 7,5 et 13 Hz. Le rythme alpha se manifeste lorsque la personne enregistrée éveillée ferme les yeux et se détend.

De la même façon que précédemment, on regroupe dans un même ensemble de données, tous les signaux cérébraux dans lesquels un motif alpha a été détecté, avec les données environnementales et externes associées.

Les informations de classification peuvent être déterminées par une combinaison de données environnementales, cérébrales et, si présentes, externes.

On comprend qu'en fonction des capteurs environnementaux présents et des signaux environnementaux que ceux-ci peuvent générer, on peut extraire une grande variété d'informations de classification.

Le choix des informations de classification peut dépendre des applications finales envisagées et peut être un paramètre configurable via une interface appropriée.

Il apparaît des exemples ci-dessus que différentes technologies d'extraction d'une classe sont possibles. On peut notamment citer :
Apprentissage machine : par exemple, un tel mécanisme peut être utilisé pour la reconnaissance automatique d'un objet dans une image (via un réseau de neurones convolutif, par exemple), l'identifiant de cet objet constituant une information de classification.

Classification par méthodes statistiques ou mathématiques (par exemple, analyse de spectres audio, regroupement de géolocalisation... )

Dans une étape S7, on peut constituer un ensemble associant ces données, ou signaux, à l'information de classification extraite. Cet ensemble peut être vu comme un ensemble de classes, chaque classe regroupant les associations de signaux correspondant à l'information de classification correspondante à cette classe.

Comme il a été évoqué, l'extraction des informations de classification constitue un étiquetage automatique des associations de signaux cérébraux / environnementaux (et externes).

Cet ensemble peut constituer un ensemble d'apprentissage pour un modèle prédictif basé sur l'apprentissage machine, tel qu'un réseau de neurones.

Selon un mode de réalisation de l'invention, on peut ainsi prévoir une étape d'entraînement S8 d'un réseau de neurones sur la base de cet ensemble constitué à l'étape S7.

En effet, connaissant pour chaque couple de signaux capturés une classe extraite, il est possible d'automatiquement effectuer un apprentissage supervisé d'un réseau de neurones en fournissant ce couple en entrée et avec une fonction de coût dépendant de cette classe extraite.

Selon un mode de réalisation de l'invention, donc, on obtient une labélisation automatique des données capturées par les différents capteurs. Cette caractéristique correspond à un avantage très intéressant puisqu'en effet, la constitution d'un ensemble d'apprentissage est une phase qui peut s'avérer très coûteuse et chronophage. Dans le cas d'espèce, il s'agit de recueillir un grand nombre de signaux extraits de flux de données cérébrales et leur associer, manuellement en général, une étiquette représentative de ce qu'on souhaite que le modèle prédise.

Selon un mode de réalisation, dans une étape S9, on peut prédire au moins une information de classification en fournissant en entrée du réseau de neurones au moins un « nouveau » signal extrait des données cérébrales. Ce signal est « nouveau » au sens où il n'appartient pas (en général) à l'ensemble d'apprentissage.

Une fois entraîné, le modèle prédictif est capable de généraliser les nouvelles entrées pour produire une probabilité de classe en sortie. Ces classes, désormais généralisables à des nouvelles entrées, peuvent être appelées « images mentales ».

En effet, il est possible à partir de signaux cérébraux, ou d'une combinaison de signaux cérébraux et de signaux environnementaux (et externes, le cas échéant) de déterminer l'image mentale correspondante.

Par exemple, à la seule pensée d'un chat, le procédé selon l'invention est en mesure de déterminer que l'utilisateur du dispositif pense à un chat, avec une certaine probabilité.

Plus précisément même, selon un mode de réalisation de l'invention, si des utilisateurs du dispositif regardent des chats, de façon automatique et transparente pour ceux-ci, et si un autre utilisateur pense à un chat, le procédé selon l'invention permettra de déterminer qu'il pense à un chat (même si ce dernier n'a jamais vu de chat avec le dispositif sur lui)

Il est ensuite possible à partir d'une information de classification ainsi prédite de déterminer une action à déclencher. Cette action peut être de différentes natures et dépend de l'application envisagée.

Selon les applications envisagées, différents types de réseaux de neurones peuvent être envisagés. Le choix d'un type de réseau de neurones peut influer sur certaines caractéristiques du mécanisme d'apprentissage, lors de cette étape S8.

À titre d'exemples, on peut citer les mémoires à long terme, les réseaux de neurones antagonistes génératifs, les réseaux de neurones convolutifs, etc.

La mémoire à long terme, ou LSTM pour « *Long Short-Term Memory* » en anglais, est une architecture de réseau neuronal récurrent artificiel (RNN pour « *Recurrent Neural Network* ») utilisée dans le domaine de l'apprentissage profond. Contrairement aux réseaux neuronaux acycliques (ou « *feedforward* » en anglais) standards, LSTM a des connexions de rétroaction. Il peut traiter non seulement des points de données uniques (tels que des images), mais également des séquences entières de données (telles que la parole ou la vidéo).

Ce type de réseaux de neurones est notamment décrit dans la page Wikipedia suivante : https://en.wikipedia.org/wiki/Long_short-term_memory, ou bien encore dans la thèse de Félix Gers, « Long short-term memory in recurrent neural networks », université de Lausanne, mai 2001, DOI : 10.5075/epfl-thesis-2366

En apprentissage, on fournit au réseau de neurones LSTM les signaux extraits sous la forme de séries temporelles (c'est-à-dire typiquement une suite de valeurs électriques exprimées en microvolts.)

On peut utiliser un tel type de réseau de neurones de différentes façons.

Par exemple, on peut fournir en entrée un signal extrait des données cérébrales sous la forme d'une série temporelle. Le réseau peut alors prédire l'appartenance de ce signal à l'ensemble d'apprentissage (cette sortie est donc booléenne).

On peut aussi fournir en entrée une association entre des signaux extraits des données cérébrales et contextuelles. Auquel cas, le réseau de neurones peut prédire une information de classification.

Un tel type de réseau de neurones peut être utilisé pour permettre de constituer une signature dématérialisée mentale d'un utilisateur du dispositif 1. Cette signature mentale pour être utilisée de la même façon qu'une signature électronique ou manuelle, afin de certifier et sécuriser une action de l'utilisateur, notamment une transaction de valeur monétaire : transfert d'un titre, transaction bancaire, etc.

Dans une phase d'apprentissage, l'utilisateur enregistre sa signature mentale. Celle-ci doit être unique et difficile à imiter (notamment à falsifier) puisqu'elle vise à valider son identité auprès des tiers et d'apposer son consentement pour certaines transactions.

Une application peut guider l'utilisateur au moyen d'une interface homme-machine afin de lui demander de penser à une succession d'éléments aussi distincts que possibles (c'est-à-dire sans corrélation décelable par un tiers). Par exemple, l'utilisateur pense, successivement, à l'image de son chat qui se repose sur un muret en Corse, à son chiffre porte-bonheur, le 3, à la côte d'agneau préparée au barbecue tous les mercredis par sa grand-mère, puis, enfin, à l'odeur de l'essence qu'il aime tant.

Pendant qu'il enregistre ces 4 éléments, l'application discrimine dans ses ondes cérébrales et dans son comportement, les caractéristiques de chaque souvenir. Les caractéristiques de chaque élément vont servir de contraintes à la création d'un modèle pour un classificateur algorithmique. Le modèle ainsi constitué (et exporté) constitue la signature mentale de l'utilisateur.

La signature ainsi exportée peut être enregistrée, ou stockée, sur un compte de l'utilisateur. Ce stockage peut être local sur le dispositif ou bien déporté afin, notamment, de pouvoir être utilisé avec d'autres dispositifs (mais le même utilisateur).

Cette signature instanciée par le modèle constitué par le réseau de neurones LSTM, peut être utilisée pour signer un nouvel acte, tout en s'assurant que l'utilisateur est bien celui qui a enregistré la signature initialement.

Pour ce faire, l'utilisateur doit se remémorer la succession de ces éléments. Le réseau de neurones de type LSTM peut alors fournir une valeur booléenne indiquant si les signaux cérébraux issus de cette remémorisation correspondent à un des exemples de l'ensemble d'apprentissage, c'est-à-dire à la signature préalablement enregistrée. La capacité de généralisation du réseau de neurones permet d'admettre une flexibilité dans la ressemblance des ondes cérébrales.

L'application peut alors refuser de poursuivre la transaction entreprise si la signature mentale ne correspond pas à celle préalablement enregistrée.

Un autre type de réseau de neurones utilisable est les réseaux de neurones antagonistes génératifs, ou GAN pour « *Generative Adversarial Network* » en anglais. Ces algorithmes ont été introduits par Ian J. Goodfellow, Jean Pouget-Abadie, Mehdi Mirza, Bing Xu, David Warde-Farley, Sherjil Ozair, Aaron Courville et Yoshua Bengio, « Generative Adversarial Networks », dans Advances in Neural Information Processing Systems 27, 2014. Ils permettent de générer des images avec un fort degré de réalisme.

En particulier, on peut utiliser un réseau de neurones GAN contraint (C-GAN pour « *Constrained* GAN »).

On peut entraîner un tel réseau avec des signaux extraits des données environnementales de type vidéo qui sont, en fait, des successions d'images numériques.

Ainsi, dans ce mode de réalisation, lors de la phase d'entraînement, on fournit une série d'images (issue d'une caméra vidéo du dispositif 1) associée à un signal extrait des données cérébrales et d'éventuelles autres données environnementales. On peut ensuite utiliser ce réseau GAN ainsi entraîné pour prédire, à partir d'un nouveau signal cérébral et d'éventuelles données environnementales associées, une nouvelle image. Cette image n'appartient pas à l'ensemble d'entraînement et est donc une création du réseau de neurones à partir des données fournies.

Cette technologie peut être exploitée de différentes façons par des applications utilisant le dispositif 1 d'analyse automatique d'ondes cérébrales.

Par exemple, une application possible consiste à mettre en image les rêves des utilisateurs.

Pour ce faire, l'utilisateur doit porter un dispositif tel que précédemment décrit, afin d'engranger des associations entre données cérébrales et données environnementales (visuelles, sonores...), notamment le jour. Au fil du temps, une vaste bibliothèque d'associations est ainsi constituée : personnes, paysages, bâtiments, ambiances... sont classifiés et à chacun de ces éléments labélisés est associé les données cérébrales correspondantes (ainsi que précédemment décrit). Un modèle peut ainsi être généré pour chaque élément vécu par l'utilisateur.

Une fois cette phase d'apprentissage réalisée, qui peut s'étendre sur plusieurs jours, l'utilisateur peut alors utiliser le dispositif pour générer de nouvelles données environnementales (visuelles, sonores...) à partir des données cérébrales.

Ces données environnementales peuvent être utilisées pour générer un médium via une interface homme-machine, par exemple une image sur un écran, un son sur un haut-parleur, etc.

Notamment, il peut porter le dispositif la nuit afin que ces nouvelles données environnementales soient générées sur la base de ses rêves. Ainsi, si l'utilisateur rêve d'un moment d'une journée passée, l'onde cérébrale devrait comporter des caractéristiques communes permettant au réseau GAN de générer des données environnementales correspondantes.

Une utilisation prolongée permet d'augmenter la « bibliothèque » de référence et d'améliorer les résultats car la probabilité de revivre en rêve une situation vécue augmente. De même, la collection de mémoires de rêves augmente jour après jour, donnant à l'utilisateur un moyen tangible de se souvenir d'événements marquants de ses nuits.

Une autre application possible du même mécanisme peut concerner un utilisateur tombé dans le coma.

Cet utilisateur utilisait, avant préalablement, un dispositif selon l'invention.

Après son hospitalisation, on lui fait porter le dispositif afin de permettre la génération, en temps-réel, de données environnementales associées à ses pensées. Par exemple, les images associées à ses pensées et générées par le réseau de neurones GAN sont affichées sur un écran.

Ces images (ou autres données environnementales) peuvent être utilisées par la famille de l'utilisateur pour tenter de comprendre l'état mental de l'utilisateur. Elles peuvent aussi permettre de voir, en temps réel, si leurs mots et gestes ont une incidence sur la génération des images et d'envisager ainsi une communication. Également, les médecins peuvent ainsi avoir un moyen pour comprendre si les traitements administrés modifient les états mentaux de l'utilisateur dans le coma.

Un autre exemple peut permettre à un conférencier d'avoir un retour en temps-réel sur les états mentaux de son auditoire. Au moins certaines personnes de son auditoire portent un dispositif conforme à un mode de réalisation de l'invention. Les données cérébrales mesurées sont fournies au réseau de neurones de type GAN qui produit en sortie des données environnementales, par exemple des images.

Ces images sont fournies sur un moniteur destiné au conférencier. Elles représentent ce qu'évoque, pour les auditeurs, le contenu sémantique de sa conférence.

Ce retour peut permettre au conférencier de mieux comprendre son public pour ajuster ses exemples et stimuler son auditoire.

Cette génération d'images est rendue possible grâce à la collecte préalable d'un grand nombre de données cérébrales sur de nombreux utilisateurs de dispositifs conformes à un mode de réalisation de l'invention. Ainsi, quand les auditeurs enfilent un tel dispositif pour la première fois, l'entraînement n'est pas nécessaire.

Un autre type de réseau de neurones est les réseaux de neurones multicouches convolutifs, ou CNN pour « *Convolutional Neural Networks* » en anglais. Ce type de réseau de neurones est largement décrit dans la littérature et fait l'objet de multiples variantes. On peut par exemple se rapporter à la page Wikipedia correspondante :
https://fr.wikipedia.org/wiki/R%C3%A9seau_neuronal_convolutif, ou bien à un ouvrage généraliste tel que « Deep Learning » de Ian Goodfellow, Yoshua Bengio et Aaron Courville, MIT Presse, 2016, ISBN 9780262035613.

On peut utiliser un tel réseau en fournissant en entrée, lors d'une phase d'apprentissage, une série d'images de la représentation des données cérébrales sous forme de spectrogrammes, par exemple. Cette image de spectrogramme peut être générée en agrégeant les données issues de différents capteurs d'ondes cérébrales 11, 12, sur une fenêtre temporelle donnée (typiquement entre 1 seconde et 10 secondes).

Une fois entraîné, le réseau de neurones convolutif peut prédire une classe lorsqu'on lui soumet un nouveau spectrogramme, ou une série de classe associée à une probabilité. Par exemple, en fournissant en entrée un spectrogramme d'un utilisateur qui regarde, ou pense, à un animal, on peut obtenir en sortie des associations classe/probabilité du type : Chat à 68%, chien à 27%, pigeon à 5% (l'ensemble des classes ainsi prédites formant une partition à 100%).

Un exemple d'application d'un tel réseau de neurones peut être le contrôle d'équipements connectés, notamment un contexte de domotique. Le dispositif, selon un mode de réalisation de l'invention, permet à son utilisateur de piloter la domotique de son logement par la seule pensée.

Dans un premier temps, l'utilisateur doit configurer son système en entraînant le réseau de neurones de type CNN.

Pour ce faire, une application logicielle peut être prévue pour guider cette phase d'entraînement. Celle-ci peut demander à l'utilisateur d'imaginer chaque commande pour chaque équipement qu'il souhaite piloter. Ainsi, le réseau de neurones peut modéliser l'association entre les ondes cérébrales correspondant à l' « imagination » de cette commande et la commande réelle de l'équipement. Ce modèle pourra ensuite être utilisé pour le pilotage effectif en utilisant le réseau de neurones en prédiction.

Ce mécanisme peut être très utile comme gain de confort, mais aussi en situation de handicap, puisque seule la pensée permet de commander une grande variété d'équipements de l'environnement de l'utilisateur.

Par exemple, un utilisateur est en situation de handicap moteur. Il a besoin d'être le plus autonome chez lui pour utiliser ses différents appareils. Sa maison est en grande partie connectée : télévision, électroménager, stores, ... Beaucoup de ses appareils sont reliés au réseau local de sa maison et son pilotables grâce à la norme MATTER que les fabricants de ces différents appareils connectés prennent en compte.

L'utilisateur commence par enfiler le dispositif et lance la configuration de la commande pour ouvrir et fermer les stores de sa maison. Pour cela, il veut imaginer un store se fermer et s'ouvrir pour les commander. L'application lui demande d'imaginer plusieurs fois la commande pour ouvrir les stores. À son tour, l'utilisateur imagine un store s'ouvrir et il fait de même pour la commande de fermeture. Une fois ces deux premières commandes enregistrées, l'application lui demande de les essayer pour continuer à apprendre et affiner le modèle de détection de l'image mentale du store qui s'ouvre et qui se ferme. L'utilisateur s'exécute. Il se déplace dans le salon, regarde le store et l'imagine en train de se fermer. C'est alors que tous les stores de la maison se ferment. Il fait de même pour la commande d'ouverture. Il peut maintenant configurer toutes les commandes une à une de tous ses appareils.

La procédure d'enregistrement des commandes est longue mais c'est un gain de temps au quotidien. En effet, l'utilisateur n'a plus besoin de se déplacer ou de toucher le bouton d'une télécommande pour effectuer des actions sur sa domotique. Il peut maintenant en permanence prendre le contrôle de sa maison. Il peut même activer l'option d'itinérance pour garder le contrôle de sa domotique même en dehors de son domicile. Ainsi, avant de rentrer chez lui, il peut allumer le chauffage uniquement par la pensée.

Un autre exemple d'application est le contrôle d'une prothèse, d'un fauteuil roulant ou d'un exosquelette pour une personne en situation de handicap moteur.

Dans l'exemple, un utilisateur est tétraplégique et se déplace en fauteuil roulant. Le contrôle de son fauteuil est fatigant. En effet, il lui demande de se saisir d'un joystick et de le déplacer dans la direction dans laquelle il souhaite se rendre.

Pour simplifier le contrôle de son fauteuil, il s'est alors doté d'un dispositif selon un mode de réalisation de l'invention. Il va configurer son fauteuil pour être contrôlé par les commandes du dispositif. Pour diriger le fauteuil, il va devoir configurer différentes images mentales correspondant à différentes actions de son fauteuil : tourner à gauche à droite, avancer, reculer, ... Chaque action correspondra à une image mentale qu'il va devoir entraîner pour que l'algorithme de classification les reconnaisse.

Pour déclencher la marche avant, il choisit d'imaginer son fauteuil en mouvement de son point de vue. Dès que l'application lui demande, il imagine son fauteuil en train d'avancer. Il fait de même pour les autres actions.

Pour s'amuser, il décide d'ajouter une deuxième commande sur la marche avant. Il veut que toutes les fois où il pense à une formule 1 rouge, son fauteuil avance aussi. Il ajoute alors une image mentale supplémentaire, comme lui demande l'application. La gamification des commandes lui permet de configurer son fauteuil comme un jeu et de passer un bon moment pendant la configuration.

Dès lors que son fauteuil est initialisé avec les images mentales, il peut se déplacer uniquement en imaginant les déplacements de son fauteuil. À tous moments, il peut ajouter de nouvelles images mentales sur des commandes existantes ou supprimer toutes les commandes et recommencer.

Bien entendu, la présente invention n'est pas limitée aux exemples et au mode de réalisation décrits et représentés, mais est définie par les revendications. Elle est notamment susceptible de nombreuses variantes accessibles à l'homme de l'art.

## Revendications

1. Dispositif (1) d'analyse automatique d'ondes cérébrales, configuré pour être porté par un utilisateur, comportant
au moins un capteur d'ondes cérébrales (11, 12), fournissant un flux (21, 22) de données cérébrales associées audit utilisateur ;
au moins un capteur environnemental (13, 14), fournissant un flux (23, 24) de données environnementales ;
des moyens (15) pour sélectionner un signal extrait dudit flux de données cérébrales, pour les associer à au moins un signal correspondant extrait dudit flux de données environnementales, pour mémoriser lesdits signaux et pour les transmettre vers un organe de traitement déporté.

2. Dispositif selon la revendication 1, dans lequel ledit au moins un capteur environnemental comprend une caméra vidéo, orientée de sorte à capturer une zone de l'espace correspondant substantiellement à celle capturée par le regard dudit utilisateur.

3. Dispositif selon l'une des revendications 1 ou 2, dans lequel ledit au moins un capteur environnemental comprend un capteur sonore.

4. Dispositif selon l'une des revendications précédentes, comportant en outre un serre-tête prévu pour maintenir ledit dispositif en place sur la tête dudit utilisateur et pour maintenir lesdits capteurs d'ondes cérébrales en contact ou à proche vicinité de ladite tête ; et un bloc d'alimentation permettant le fonctionnement desdits capteurs et desdits moyens.

5. Dispositif selon l'une des revendications précédentes, configuré pour associer auxdits signaux un horodatage et/ou un identifiant dudit dispositif.

6. Dispositif selon l'une des revendications précédentes, dans lequel la localisation d'un capteur cérébral dépend d'un type dudit au moins capteur environnemental.

7. Dispositif selon l'une des revendications précédentes, comportant en outre des moyens pour acquérir un flux de données externes provenant d'une source extérieure audit dispositif, et pour associer ledit signal extrait dudit flux de données cérébrales à au moins un signal correspondant extrait dudit flux de données externes.

8. Système comportant un ou plusieurs dispositifs selon l'une des revendications précédentes, et un organe de traitement déporté configuré pour mémoriser l'ensemble des signaux provenant dudit un ou plusieurs dispositifs.

9. Système selon la revendication précédente, comportant des moyens pour extraire une information de classification à partir desdites données, et pour constituer un ensemble associant lesdites données à l'information de classification extraite.

10. Système selon la revendication précédente, comportant des moyens pour entraîner un réseau de neurones sur la base dudit ensemble.

11. Procédé d'analyse automatique d'ondes cérébrales à l'aide d'un dispositif configuré pour être porté par un utilisateur, le procédé comportant des étapes de
génération (S1) d'un flux de données cérébrales associées audit utilisateur au moyen d'au moins un capteur d'ondes cérébrales ;
génération (S2) d'un flux de données environnementales au moyens d'au moins un capteur environnemental ;
association (S3) d'un signal sélectionné extrait dudit flux de données cérébrales à au moins un signal correspondant extrait dudit flux de données environnementales ;
mémorisation (S4) desdits signaux et transmission (S5) vers un organe de traitement déporté.

12. Procédé selon la revendication précédente, comportant en outre une étape d'extraction (S6) d'une information de classification à partir desdites données, et une étape de constitution (S7) d'un ensemble associant lesdites données à l'information de classification extraite.

13. Procédé selon la revendication précédente, comportant en outre une étape d'entraînement (S8) d'un réseau de neurones sur la base dudit ensemble.

14. Procédé selon l'une des revendications 11 à 13, comportant une étape de prédiction (S9) d'une information de classification à partir d'au moins un nouveau signal extrait des données cérébrales.

15. Programme d'ordinateur comportant des instructions pour mettre en œuvre le procédé selon l'une quelconque des revendications 11 à 14 lorsqu'exécuté sur une ou plusieurs plateformes de traitement de l'information.

## Revendications modifiées

### Revendications modifiées conformément à la règle 137(2) CBE.

1. Système comportant un ou plusieurs dispositifs (1) d'analyse automatique d'ondes cérébrales, configuré pour être porté par un utilisateur, et un organe de traitement déporté configuré pour mémoriser l'ensemble des signaux provenant dudit un ou plusieurs dispositifs, ledit un ou plusieurs dispositifs comportant
au moins un capteur d'ondes cérébrales (11, 12), fournissant un flux (21, 22) de données cérébrales associées audit utilisateur ;
au moins un capteur environnemental (13, 14), fournissant un flux (23, 24) de données environnementales ;
des moyens (15) pour sélectionner un signal extrait dudit flux de données cérébrales, pour les associer à au moins un signal correspondant extrait dudit flux de données environnementales, pour mémoriser lesdits signaux et pour les transmettre vers ledit organe de traitement déporté, et
ledit organe de traitement déporté comportant des moyens pour extraire une information de classification à partir desdites données, et pour constituer un ensemble associant lesdites données à l'information de classification extraite.

2. Système selon la revendication 1, dans lequel ledit au moins un capteur environnemental comprend une caméra vidéo, orientée de sorte à capturer une zone de l'espace correspondant substantiellement à celle capturée par le regard dudit utilisateur.

3. Système selon l'une des revendications 1 ou 2, dans lequel ledit au moins un capteur environnemental comprend un capteur sonore.

4. Système selon l'une des revendications précédentes, comportant en outre un serre-tête prévu pour maintenir ledit dispositif en place sur la tête dudit utilisateur et pour maintenir lesdits capteurs d'ondes cérébrales en contact ou à proche vicinité de ladite tête ; et un bloc d'alimentation permettant le fonctionnement desdits capteurs et desdits moyens.

5. Système selon l'une des revendications précédentes, configuré pour associer auxdits signaux un horodatage et/ou un identifiant dudit dispositif.

6. Système selon l'une des revendications précédentes, dans lequel la localisation d'un capteur cérébral dépend d'un type dudit au moins capteur environnemental.

7. Système selon l'une des revendications précédentes, comportant en outre des moyens pour acquérir un flux de données externes provenant d'une source extérieure audit dispositif, et pour associer ledit signal extrait dudit flux de données cérébrales à au moins un signal correspondant extrait dudit flux de données externes.

8. Système selon l'une des revendications précédentes, comportant des moyens pour entraîner un réseau de neurones sur la base dudit ensemble.

9. Procédé d'analyse automatique d'ondes cérébrales à l'aide d'un dispositif configuré pour être porté par un utilisateur, le procédé comportant des étapes de
génération (S1) d'un flux de données cérébrales associées audit utilisateur au moyen d'au moins un capteur d'ondes cérébrales ;
génération (S2) d'un flux de données environnementales au moyens d'au moins un capteur environnemental ;
association (S3) d'un signal sélectionné extrait dudit flux de données cérébrales à au moins un signal correspondant extrait dudit flux de données environnementales ;
mémorisation (S4) desdits signaux et transmission (S5) vers un organe de traitement déporté ;
extraction (S6), par ledit organe de traitement déporté, d'une information de classification à partir desdites données, et constitution (S7) d'un ensemble associant lesdites données à l'information de classification extraite .

10. Procédé selon la revendication précédente, comportant en outre une étape d'entraînement (S8) d'un réseau de neurones sur la base dudit ensemble.

11. Procédé selon l'une des revendications 9 à 10, comportant une étape de prédiction (S9) d'une information de classification à partir d'au moins un nouveau signal extrait des données cérébrales.

12. Programme d'ordinateur comportant des instructions pour mettre en oeuvre le procédé selon l'une quelconque des revendications 9 à 11 lorsqu'exécuté sur une ou plusieurs plateformes de traitement de l'information.
